Europäisches Patentamt

European Patent Office

Office européen des brevets

(11) Publication number: **0 046 783**
**B1**

# EUROPEAN PATENT SPECIFICATION

(45) Date of publication of patent specification: **30.01.85**

(21) Application number: **81900572.9**

(22) Date of filing: **19.02.81**

(86) International application number:
**PCT/DK81/00018**

(87) International publication number:
**WO 81/02467 03.09.81 Gazette 81/21**

(51) Int. Cl.⁴: **G 01 N 21/17, G 01 N 21/35,
G 01 N 33/02**

(54) A METHOD FOR QUANTITATIVELY DETERMINING COMPONENTS IN A SAMPLE.

(30) Priority: **19.02.80 DK 723/80**

(43) Date of publication of application:
**10.03.82 Bulletin 82/10**

(45) Publication of the grant of the patent:
**30.01.85 Bulletin 85/05**

(84) Designated Contracting States:
**DE FR GB NL SE**

(56) References cited:
**EP-A-0 012 492
WO-A-80/02597
DE-A-2 538 145
GB-A- 989 617
GB-A-1 559 792
GB-A-2 008 745
US-A-4 239 394**

(73) Proprietor: **A/S N. Foss Electric
Slangerupgade 69
DK-3400 Hilleroed (DK)**

(72) Inventor: **BJARNO, Ole-Christian
Pilekaeret 6
DK-2840 Holte (DK)**

(74) Representative: **Smith, Sydney et al
Elkington and Fife High Holborn House 52/54
High Holborn
London WC1V 6SH (GB)**

Courier Press, Leamington Spa, England.

## Description

There is a great demand for quantitatively determining the content of fat and protein in foodstuffs and animal feeds and in raw materials for making foodstuffs and animal feeds.

Until a few years ago, the completely dominating method for protein determination was the Kjeldahl method (e.g., in a modified automated form, cf. Poul Erik Ægidius, Analytical Apparatus for Serial Determination of Nitrogen in Samples by the Kjeldahl Method, US Patent US—A—3,964,869), but also colour-binding (The Pro-Meter, the Bakers Digest, December 1974, p. 28—30) and the biuret reaction (Alvin J. Pinckney, Wheat Protein and the Biuret Reaction, Cereal Chemistry, Vol. 26, November 1949, No. 6) had gained a certain acceptance, but only for the determination of dissolved proteins, for example for determining protein in milk and serum (George R. Kingsley, The Determination of Serum Total Protein, Albumine, and Globumine by the Biuret Reaction, J. Biol. Chem., Vol. 131, 1939, p. 197).

In the same manner, the dominating method for fat determination has been extraction with organic solvents and determination, on the clear extract, of a physical property indicative of the fat content, such as dielectricity constant, refractive index, or density, for example in automated form in "Foss-Let" (Poul Erik Ægidius, Apparatus for Density Determination of a Liquid, US Patent US—A—3,815,424). (However, for special purposes, such as fat determination in milk, measurements by light scattering have been widely used (Poul Erik Ægidius, US Patent US—A—3,442,623)).

Both of the dominating methods (Kjeldahl and extraction) suffer from the disadvantage that they are difficult to perform, use poisonous chemicals, and yield poisonous waste fluids. Both biuret and colour-binding suffer from the disadvantage that the calibration is strongly dependent upon which product is to be determined. Therefore, it was a technological break-through when Goulden (J.D.S. Goulden, Infrared Spectroscopy of Diary Products, J. Sci. Food Agric., 7, September 1956) showed that it was possible to quantitatively measure protein and fat in milk by means of infrared (IR) photometry. This had until then been considered impossible due to the strong water bands, but when Goulden published his work, the photometric technology had been developed so far that it was then possible to compensate effectively for interferences.

Goulden's discovery formed the basis of several IR transmission instruments for routine analyses in milk and milk products (cf. a thesis by John Shields submitted for the degree of Bachelor of Philosophy at the University of York, November 1975, Sten Andersen Nexø and Andreas Skærlund Frandsen, An Apparatus for Measuring Components of Liquid Samples, US Patent Application US—A—4236075, Japanese Patent Application JP—A—53—111829, British Patent Application GB—A—2028498 and German Patent Application DE—A—28 38 706; Sten Andersen Nexø and Henrik Rastrup Andersen, Method for Quantitatively Determining Fat in a Fat Containing Sample, European Patent Application EP—A—0012492 which was filed before but published after the priority date of the present application; Milco-Scan 104 19900 applications guide, issues of March 1979 and November 1979, A/S N. Foss Electric, Hillerød, Denmark).

The use of infrared technology for solid products has also been introduced (Donald R. Webster and Eugene R. Gansell, Optical Internal Quality Analyzer, U.S. Patent No. 3,765,775, C. A. Watson Near Infrared Reflectance Spectrofotometric Analysis of Agricultural Products, Analytical Chemistry, Vol. 49, No. 9, August 1977, p. 835a). The measurement was in this case, of course, limited to the measurement of reflected light from the surface of a finely ground sample. Also, it was necessary to operate in the near infrared range to obtain sufficient energy, which necessitated the use of the less sharp overtones of the fundamental IR bands for protein and fat. This fact, in connection with the high sensitivity of the method to variations in the sample surface, resulted in a less than satisfactory precision (R. Sepp, Starch, 31, 1979, p. 57—63, Rapid Protein Determination), but the method is relatively fast and does not make use of poisonous chemicals. However, there exists a great demand for a *precise* method for determination of several essential components, typically two or more components, especially fat and protein, in various "difficult" products such as animal feed mixtures and raw materials therefor, typically cereals, oil-containing seeds and animal residual products, as well as for a satisfactory and safe, precise determination of fat and protein in a great variety of food products which are not milk-based. In the present context, the term "not milk-based" is intended to indicate products which are not milk products and do not contain predominant amounts of milk products. Thus, products which are "not milk-based" exclude such products as skim milk, ice-cream, yoghurt, ymer, cheese, and corresponding products which imitate such products such as ice-creams not based on milk fat, etc. It is well known that minor amounts of milk products such as skim milk powder, whey protein concentrate, etc. are added to other food products, for example in order to increase their protein content or render the products cheaper. However, provided that the product with such addition comprises a predominant amount of not milk-based constituents, typically at least 80% and often at least 90%, based upon dry matter, such products with minor added milk-based constituents will be considered "not milk-based" products in the present context, considering that the predominant components thereof are not milk-based. Typical examples of not milk-based food products are meat and meat products such as bacon, beef, sausage products, ham, pork, mutton, horse meat, whale meat, poultry such as geese, ducks, turkeys and chicken, goat meat, minced meat, meat-based baby food,

2

**0 046 783**

and meat-based or meat-containing ready-made dishes. The greatest demand for a determination of the above type is in the field of processed meat products such as sausages, hamburgers, luncheon meat, pastes such as liver paste, pies and patties. In this context, a precise method is intended to designate a method having a precision of the same level as the standard methods, which means 1—2% relative.

It has now been found that the content of at least two chemical constituents, typically fat and protein, in a solid or semisolid sample of a protein containing non-milk based product, typically a sample of a material selected from the group consisting of foodstuffs, animal feeds and raw materials for foodstuffs and animal feeds, and ready-prepared dishes, can be quantitatively determined without the use of complicated methods or hazardous chemicals and with surprising precision by a method which according to the invention comprises converting the sample into dispersed form by treatment with a dispersing reagent combined with mechanical subdivision to obtain a dispersion in which the protein content is up to 20% by weight and in which the chemical constituents can be determined by quantitative photometric infrared absorption determination, determining the content of the constituents in the dispersion by such photometric determination, and correlating the determination so obtained to a quantitative determination of the constituents as assayed by a reference method.

The above-mentioned European patent application published under number 0012492, which was not available to the public on the priority date of the present application, discloses a particular method for the quantitative determination of fat in a fat-containing sample, in particular milk or a milk product. Although it is mentioned that this particular method may also be performed on suitably prepared samples of meat, the method is mainly aimed at the exact determination of only one component, namely fat. It is mentioned that in a preferred embodiment, protein is also measured, but this measurement is only of a secondary nature as it is intended as an auxiliary measurement in order to aid in correcting the fat determination by compensating for the influence of any protein present. Nor is there in the European Application any specification of any protein content range within which such a protein determination could be conducted.

As mentioned above, IR transmission photometry has been successfully developed into a most useful method for quantitative determination of protein and fat in milk and milk products.

However, it has now surprisingly been found that by the combination of treating with a dispersing reagent, mechanically subdividing, performing a quantitative infrared transmission photometric determination on the resulting dispersion, and correlating the quantitative determination so obtained to quantitative determination performed by any accepted method, preferably the standard methods, of the constituents to be determined, samples of a completely different character from milk and milk products, and even samples of a highly complex and difficulty dispersible character can be quantitatively determined with respect to the constituents in question, typically fat and protein with an excellent degree of exactitude which, in spite of the fact that the constituents of the sample are not in all cases rendered available to the determination in question in the ordinary sense of this term, and in spite of the fact that the dispersion may contain a high number of components in different types of systems such as in solution, emulsion, and suspension, reaches or even surpasses the exactitude and reproducibility of the standard methods, but without the evident drawbacks and hazards of the standard methods.

In the method of the present invention, the combination of the treatment of the sample with the dispersing reagent and the mechanical subdivision aims at producing, from the sample, a dispersion having such a small maximum particle size that the dispersion can be subjected to the IR transmission photometry determination. The maximum particle size of the dispersion should be below about 6 $\mu$m, and the preferred maximum particle size of the dispersion is 1 $\mu$m or less. The term "dispersion" is used in the present context to designate any combination of "solution", "emulsion", and "suspension". It is one of the main features of the present invention that the dispersion subjected to the photometric determination will typically be a combination of a solution, an emulsion, and a suspension, and the remarkable fact is that such multi-phase, multi-component system generated from the solid sample in question will give rise to quantitative determination of several constituents of the original solid sample with exact and reproducible correlation to the true value of the particular constituent as determined by one of the standard methods.

The mechanical subdivision of the sample is suitably performed by mechanically treating a slurry of particles of the sample in the dispersing reagent with suitable subdividing means. The starting slurry may be obtained by first coarsely dividing the sample, if necessary (e.g., if the sample is constituted by coherent pieces such as e.g. meat), and adding a suitable amount of dispersing reagent to the sample. The slurry will typically have a solids content of at the most 30 g per 100 ml, often 2 to 20 g per 100 ml. A particular type of mechanical subdividing treatment which has been found to result in a particularly good dispersion of the samples is treatment in a grinder of the type having a closed chamber in which an impact means is slidably mounted for substantially reciprocating movement of a predetermined stroke length, especially a particular version of such a grinder described in US patent US—A—3,672,659, that is, a grinder having a closed cylindrical chamber in which a smooth cylindrical body of substantially smaller diameter than the chamber is slidably mounted for substantially reciprocating movement of a predetermined stroke length, and suitable conditions and

3

parameters of this wet mechanical subdivision treatment are, for the various types of cereals, animal feeds, meat, and other types of like products, a weight of the cylindrical body of about 400 g, a stroke length of about 5 mm, a stroke frequency of about 3000 per minute, and a treatment time of about 4 minutes. These parameters and conditions may, of course, be modified to suit a particular type of sample to be treated, and the particular type of dispersing reagent used. Within the scope of the present invention, any other mechanical wet subdividing treatment substantially corresponding to the abovementioned special treatment with respect to the effect thereof can also be utilized.

The combination of the treatment with a dispersing agent and the mechanical subdivision will either in itself convert the sample into a dispersed form which will be suitable for the photometric quantitative determination, or a separation of particles which are of a size that would disturb the photometric measurement will have to be performed prior to the measurement. It is preferred to avoid such separation of any component from the dispersion, and for this reason, it has been found suitable, with products of a difficult character, for example, products containing proteins of a complex and robust character, such as shell proteins, hard cereals, etc., to perform a mechanical dry pre-grinding as part of the mechanical subdivision, to yield particles in the desired particle size range prior to the wet mechanical treatment in the presence of the dispersing reagent.

As mentioned above, a separation, from the dispersion, of particles which are of a too large size for the photometric determination should preferably be avoided by securing that the dispersion, after the wet treatment, does not contain such particles. The photometric measurement is then performed on the complete sample, and this in itself contributes to the best exactitude and reproducibility; furthermore, the photometric determination, e.g. the IR transmission photometry, yields the highest signal to noise ratio when the concentration of the constituents to be determined, such as protein and fat, is sufficiently high. However, if a separation is in fact performed, it should preferably be limited so that at any rate at least 50% of the content of the constituents to be determined, e.g., the content of fat and protein, are allowed to remain in the dispersion after the separation, and preferably, the separation will remove at the most 10%, more preferably at the most 5%, of the constituents to be determined. Separation, when performed, may, e.g., be performed by centrifugation, typically to an extent corresponding to about 2000 g $\times$ 10 min, or by filtration, typically through a filter allowing passage of particles of at the most 5 $\mu$m.

The dispersing reagents which are used in the method of the present invention are typically aqueous solutions, especially aqueous solutions which are of a basic character.

In the present context, the term "basic" is intended to designate a reagent which has a pH that is above 7, typically above 9 and examples of useful basic reagents are alkaline solutions which have a pH of at least 10, typically a pH in the range of 12—14. Such solutions, e.g. sodium hydroxide solution having a concentration of about 0.5 M, exert a dispersing and emulsifying activity on fat and protein-containing samples at temperatures in a suitable range, which is at least about 40°C, e.g. 40—60°C, such as 45—55°C. Such a temperature will usually inherently be obtained in the abovementioned mechanical treatment. Addition of an emulsifier to the solution will usually enhance its capability of yielding the desired degree of dispersion and may thus be used to lower the necessary pH of a particular basic dispersing reagent.

Dispersing reagents of types which are complex-forming with proteins have been found result in an efficient dispersion of the samples for the purpose of the present invention. Typical examples of such complex-forming reagents are basic copper-containing solutions such as a basic solution of a complex of copper with a component selected from the group consisting of amines, ammonia, tartrates, and ethylene glycol, but also complex-forming basic reagents which are basic solutions containing a protein-dissolving complex selected from the group consisting of nickel complexes, mercury complexes, cadmium complexes, zinc complexes, and cobalt complexes, are useful dispersing agents for the purpose of the present invention. Also these types of dispersing agent will preferably contain an emulsifier. For safety reasons, it will often be preferred to avoid the use such complex-forming reagent when possible, as they contain metals which are mostly of a character considered undesirable for incorporation in a routinely used reagent.

A preferred type of apparatus for performing the photometric determination is an IR transmission photometry instrument of the same type as is used for routine measurements on milk, such as a "Milko-Scan" instrument (cf., e.g., "Milko-Scan 102/103/104, Operating Instructions", A/S N. Foss Electric, Hillerød, Denmark, 1979 and the patent applications mentioned on page 2 of the present specification. These instruments and instruments of similar types are adapted to measure fat, protein, and lactose in milk and may either use the conventional carbonyl band for the fat determination or may (European Patent Application No. 79.301.465.5) use, as sample wavelength for the fat determination, a band characteristic to carbon-hydrogen bonds, in particular a sample wavelength in the interval from 3.475 to 3.41 $\mu$m and a reference wavelength for the fat determination in the range between 3.51 and 3.60 $\mu$m.

In order to correlate the results obtained in the quantitative photometric determinations on the dispersion to the authentic content, in the sample product, of the constituent in question, best line fit or any other suitable correlation is performed to determinations made on the same products using a suitable reference method, e.g. one of the standard methods. One suitable way is to refer to the Soxhlet or Foss-Let method for the fat determination and the Kjeldahl or Kjel-Foss method for the protein determination. Based upon the best line fit, the conversion function to be used for the particular product type and the particular type of apparatus is found. According to the invention, it has been found that the origin of the various product types will not give great variation of the measured results. As an example of this, it may be mentioned that feed mixtures for swine, with a very high variation in contents, cf. Example 4, will still give a high correlation to the standard methods, which means that when a multicomponent system is determined, the origin of the various proteins and other constituents therein can be varied within wide limits. The correlation becomes more sensitive when less complex products are determined, as the fact that the proteins are different and have a different molecular weight will then manifest itself to a higher degree. Thus, a general regression equation which has been used, e.g. in Example 4, and which has been found to be a useful regression equation for fat and protein determination in several types of products, was found to be unsuitable for fat and protein determination for soybeans.

The present invention provides, for the first time, an easy and exact method for hazard-free determination of fat *and* protein in a solid sample with an absolutely satisfactory exactitude and reproducibility. Also, the invention makes it possible to simultaneously measure other components in the sample, such as total carbohydrate (vide Example 8), and/or to perform other desired determinations including differential measurements such as determinations of the content of connective tissue protein in comparison with total protein (by measurement on partly a dispersion where total protein has been made available to the determination and partly a dispersion where the more difficulty dispersible connective tissue protein has not been made available), etc. Thus, it will be realized that the method of the invention opens up very important new fields and constitutes a remarkable improvement over the known art methods, including a very considerable increase of exactitude in comparison with near-infrared reflection photometry which was the only known art multicomponent method available for solid products.

Example 1

6 g of fish meal were weighed out and transferred to a Foss-Let reaction chamber (a grinder having a closed cylindrical chamber in which a smooth cylindrical body of a weight of about 400 g is slidably mounted and reciprocates at a stroke frequency of about 3000/minute at a stroke length of about 5 mm). 100 ml of a dispersing reagent consisting of 0.1 M copper sulphate, 0.5 M NaOH and 0.125 M sodium sulphite and 10% ethylene glycol were added.

The mixture was treated for 2 minutes and 4 minutes, respectively, in the reaction chamber, whereafter the resulting dispersion was centrifuged at 2000 g for 20 minutes; the centrifugate was thereafter measured on a Milko-Scan 104, and the protein content in the centrifugate and in the fish meal was also determined by Kjeldahl analysis.

On 7 different samples of fish meal, an average availability of fat and protein of 80% at a treatment time of 2 minutes and 95% at a treatment time of 4 minutes was found.

At a treatment of 4 minutes, a standard deviation between Milko-Scan 104 measurement and Kjeldahl measurement of 0.8% protein=1.5% relative was found, which is an uncertainty of the same order as the reproducibility of the standard method (Kjeldahl).

Example 2

6 g fish meal were weighed out and transferred to a reaction chamber of the same type as described in Example 1. 100 ml of dispersing reagent consisting of 0.1 M copper sulphate, 0.5 M NaOH and 2.5% KNa-tartrate were added.

The mixture was treated, centrifuged and analyzed by means of the Kjeldahl method on the protein available.

An availability degree of 96% was found.

Example 3

6 g of fish meal were weighed out and transferred to a reaction chamber of the same type as described in Example 1. 100 ml of a dispersing reagent consisting of 0.1 M copper sulphate, 0.5 M NaOH, 0.1 M ethylene diamine and 0.1 M propylene diamine were added.

An availability degree of 98% was found.

Example 4
10 swine feed mixtures were prepared as follows:

| Gram/100 g mixture | 1 | 2 | 3 | 4 | 5 | 6 | 7 | 8 | 9 | 10 |
|---|---|---|---|---|---|---|---|---|---|---|
| Fish meal | 18 | 15 | 12 | 9 | 6 | 3 | 21 | 24 | 27 | 30 |
| Meat-and-bone-meal | 3 | 6 | 9 | 12 | 15 | 18 | 3 | 3 | 3 | 3 |
| Barley | 40 | 42 | 44 | 46 | 46 | 47 | 33 | 27 | 29 | 28 |
| Soy meal | 35 | 32 | 29 | 29 | 23 | 20 | 35 | 35 | 35 | 35 |
| Fat | 4 | 5 | 6 | 7 | 10 | 12 | 8 | 11 | 6 | 4 |
| % protein | 34.25 | 32.40 | 30.61 | 28.81 | 26.82 | 29.94 | 36.43 | 37.31 | 39.66 | 41.71 |
| % fat | 6.85 | 7.88 | 8.91 | 9.98 | 12.93 | 14.94 | 10.90 | 14.97 | 9.23 | 8.41 |

6 g sample were weighed out and transferred to a reaction chamber of the same type as described in Example 1. 100 ml of a reagent consisting of 0.1 M copper sulphate, 0.5 M NaOH, 0.1 M ethylene diamine and 0.1 M propylene diamine were added.

The mixture was treated for 4 minutes, centrifuged for 10 minutes, and the centrifugate was thereafter measured on a Milko-Scan 104. All measurements were repeated three times to obtain an estimate of the reproducibility.

Protein:

| | |
|---|---|
| Correlation coefficient | 0.997 |
| Reproducibility, standard deviation | 1.8% rel. |
| Intermethod standard deviation | 1.1% rel. |

Fat:

| | |
|---|---|
| Correlation coefficient | 0.996 |
| Reproducibility, standard deviation | 1.7% rel. |
| Intermethod standard deviation | 2.1% rel. |

Example 5

A swine feed mixture consisting of:

| Gram/100 g mixture | | |
|---|---|---|
| | Lard | 6 |
| | Barley | 36 |
| | Oat | 14 |
| | Wheat | 14 |
| | Soy meal | 9 |
| | Skim milk powder | 9 |
| | Fish meal | 4 |
| | Dry yeast | 3 |
| | Sugar | 3 |
| | Vitamins | 2 |

8.09% Fat

was prepared          17.1% Protein

A sample was reacted as in Example 4. Subsequent to the reaction, the temperature of the reaction mixture was measured, and the loss of water during the centrifugation was measured. The test was repeated 10 times.

The total reproducibility of 1.5% rel. protein could thereafter be split up into the following standard deviations:

| | |
|---|---|
| Varying loss of water | 0.5% |
| Varying reaction temperature | 1.0% |
| Residual variation | 1.0% |

If the reaction is performed at the same temperature, the reproducibility will be about 1% rel. protein.

Example 6

The following meat products were ground through an ordinary kitchen machine and measured with triple determination on a Kjel-Foss analyser and double determination by the Foss-Let extraction determination

|  | Kjel-Foss determination mean | Foss-Let determination mean |
|---|---|---|
| Bacon | 15.2 | 39.6 |
| Beef | 22.2 | — |
| Sausage | 19.4 | 26.74 |
| Sausage | 12.4 | 21.22 |
| Sausage | 12.9 | 26.30 |
| Sausage | 18.2 | 31.78 |
| Ham | 21.9 | 9.99 |
| Salami | 13.6 | 36.47 |
| Lamb steak | 21.7 | 5.3 |
| Pork | 11.6 | 46.3 |

Thereafter, a sample of 20 g was taken out and reacted for 5 minutes in a reaction chamber of the same type as described in Example 1 with 100 ml of a dispersing reagent of the same composition as in Example 2 with a further addition of 1 ml of an emulsifier consisting of 10% "TWEEN" 20 (Atlas), 40% A 109, 25% A 431, 10% B 246, 10% B 261 and 5% A 394 (from ICI). Triple treatment (mechanical plus reagent) was performed, and each sample was measured 5 times on Milko-Scan 104 without any centrifugation.

Protein:

| Correlation | 0.993 |
|---|---|
| Intermethod variation | 2.3% rel. |
| Reproducibility | 1.5% rel. |

Fat:

| Correlation | 0.9994 |
|---|---|
| Intermethod variation | 0 |
| Reproducibility | 1.5% rel. |

Example 7

19 mixtures of pork, lard and water were prepared in such a manner that the protein content varied evenly from 11 to 20% and the fat content varied from 10 to 30%.

The samples, 10 g, were reacted for 4 minutes in a reaction chamber of the same type as described in Example 1 with 0.5 M NaOH as dispersing reagent. Each sample was reacted twice, and each treated sample was measured three times on a Milko-Scan 104, the only further intermediate treatment being thermostating in a water bath at 40°C. Multiple regression analysis was performed with fat and protein as independent variants.

Protein:

| | |
|---|---|
| Correlation | 0.999 |
| Intermethod variation | 0.25% rel. |
| Reproducibility | 1.12% rel. |

Fat:

| | |
|---|---|
| Correlation | 0.998 |
| Intermethod variation | 1.75% rel. |
| Reproducibility | 1.75% rel. |

The measuring accuracies were 10—20 fold better than those reported for meat products measured by near-infrared reflection photometry.

(Cf.: E. Hauser & V. Weber, Fleischwirtschaft, Vol. 58, p. 452—4, 457—9 (1978)).

Example 8

8 mixtures of black pudding with a fat content varying from 6.8 to 22%, a protein content from 8 to 16% and a carbohydrate content from 8 to 26% were prepared. The measurements were performed according to the standard procedure with the following results:

| Fat % calculated | Fat % MSc 104* | Protein % calculated | Protein % MSc 104* | Carbohydrate calculated | Carbohydrate MSc 104* |
|---|---|---|---|---|---|
| 22.0 | 21.2 | 8.0 | 7.9 | 26.0 | 26.3 |
| 22.0 | 22.0 | 8.0 | 8.0 | 26.0 | 26.2 |
| 20.3 | 20.2 | 10.0 | 9.9 | 24.0 | 24.2 |
| 20.3 | 20.5 | 10.0 | 9.9 | 24.0 | 24.3 |
| 18.6 | 18.9 | 12.0 | 11.8 | 22.0 | 22.1 |
| 18.6 | 18.7 | 12.0 | 11.8 | 22.0 | 22.0 |
| 16.9 | 17.2 | 14.0 | 14.5 | 10.0 | 19.5 |
| 16.9 | 17.2 | 14.0 | 13.4 | 20.0 | 20.0 |
| 15.2 | 14.9 | 16.0 | 16.2 | 18.0 | 18.0 |
| 15.2 | 15.2 | 16.0 | 16.3 | 18.0 | 17.6 |
| 13.5 | 13.8 | 18.0 | 18.4 | 16.0 | 15.6 |
| 13.5 | 13.7 | 18.0 | 18.5 | 16.0 | 15.8 |
| 11.9 | 11.9 | 20.0 | 20.3 | 14.0 | 14.1 |
| 11.9 | 12.0 | 20.0 | 19.8 | 14.0 | 13.9 |
| 6.8 | 6.7 | 26.0 | 25.8 | 8.0 | 8.3 |
| 6.8 | 6.6 | 26.0 | 25.5 | 8.0 | 8.5 |
| Correlation | 0.999 | | 0.999 | | 0.999 |
| Accuracy SD | 0.19% fat | | 0.12% protein | | 0.22% carbohydrate |
| Repeatability SD | 0.24% fat | | 0.31% protein | | 0.19% C |

*) Milko-Scan 104

From the above appears that, on a difficult product such as blood pudding, the three parameters are measured with an acceptable accuracy.

**0 046 783**

## Claims

1. A method for quantitatively determining the content of at least two chemical constituents such as fat and protein in a solid or semisolid sample of a protein-containing non-milk-based product, comprising converting the sample into dispersed form by treatment with a dispersing reagent combined with mechanical subdivision to obtain a dispersion in which the protein content is up to 20% by weight and in which the said chemical constituents can be determined by a quantitative infrared absorption photometric determination, determining the content of the constituents in the dispersion by such photometric determination, and correlating the determination so obtained to a quantitative determination of the constituents as assayed by a reference method.

2. A method as claimed in claim 1 in which the chemical constituents determined comprise fat and protein.

3. A method as claimed in any of claims 1 or 2 in which the sample is a solid or semisolid sample selected from the group consisting of foodstuffs, animal feeds and raw materials for foodstuffs and animal feeds.

4. A method as claimed in any of the preceding claims in which the mechanical treatment to finely divide the sample is performed on a slurry of the sample having a solids concentration of at the most 30 g per 100 ml, typically 2—20 g per 100 ml.

5. A method as claimed in any of the preceding claims in which the dispersion subjected to the photometric determination has a particle size of less than about 6 $\mu$m.

6. A method as claimed in claim 5 in which the particle size is about 1 $\mu$m or less.

7. A method as claimed in claim 5 or 6 in which the reduction to the maximum particle size stated is performed solely by the combination of mechanical subdivision and treatment with the dispersing reagent and without performing any separation on the dispersion.

8. A method as claimed in claim 5 or 6 in which particles which are of a size of 6 $\mu$m and above are separated from the dispersion prior to the photometric determination.

9. A method as claimed in claim 8 in which at most 10%, preferably at most 5%, of the constituent or constituents to be determined are removed by the separation.

10. A method as claimed in claim 8 or 9 in which the separation is performed by centrifugation to an extent corresponding to about 2000 g×10 min.

11. A method as claimed in claim 8 or 9 in which the separation is performed by filtration through a filter permitting passage of particles of at the most 5 $\mu$m.

12. A method as claimed in any of the preceding claims in which the mechanical treatment is performed in a grinder having a closed cylindrical chamber in which a smooth cylindrical body of substantially smaller diameter than the chamber is slidably mounted for substantially reciprocating movement of a predetermined stroke length.

13. A method as claimed in claim 12 in which the stroke length is about 5 mm, the stroke frequency is about 3000 per min, the treatment period is about 4 min, and the weight of the cylindrical body is about 400 g.

14. A method as claimed in any of the preceding claims in which the dispersing reagent is an aqueous solution.

15. A method as claimed in claim 14 in which the aqueous solution is a basic solution.

16. A method as claimed in claim 15 in which the pH of the solution is at least 9.

17. A method as claimed in claim 16 in which the pH of the solution is 12—14.

18. A method as claimed in any of the preceding claims in which the dispersing agent is an alkali hydroxide solution such as about 0.5 M NaOH solution.

19. A method as claimed in any of claims 1—13 in which the dispersing agent is a reagent which is complex-forming with proteins to form a solution or a colloid solution.

20. A method as claimed in claim 19 in which the complex-forming reagent is a basic copper-containing solution.

21. A method as claimed in claim 20 in which the basic copper solution is a basic solution of a complex of copper with a component selected from the group consisting of amines, ammonia, tartrates, and ethylene glycol.

22. A method as claimed in claim 19 in which the complex-forming basic reagent is a basic solution containing a protein-dissolving complex selected from the group consisting of nickel complexes, mercury complexes, cadmium complexes, zinc complexes, and cobalt complexes.

23. A method as claimed in any of the preceding claims in which the dispersing reagent contains an emulsifier.

24. A method as claimed in any of the preceding claims in which the quantitative determination of the content of fat and protein in the sample on the basis of the IR transmission photometry measurement is performed by relating the IR transmission reading to quantitative determinations made on the same material as constitutes the samples by means of standard methods.

25. A method as claimed in claim 24 wherein the standard method to which relation is made is the Soxhlet or Foss-Let method for the fat determination and the Kjeldahl or Kjelfoss method for the protein determination.

10

26. A method as claimed in any of the preceding claims in which the temperature of the sample when treated with the dispersing reagent is at least 40°C.

**Revendications**

1. Procédé de détermination quantitative de la teneur d'un échantillon solide ou semi-solide de produit protéiné et non laitier en au moins deux constituants chimiques tels que graisse et protéine, comprenant la transformation de l'échantillon en une forme dispersée par traitement avec un réactif de dispersion combinée à une subdivision mécanique pour obtenir une dispersion dans laquelle la teneur en protéine est inférieure ou égale à 20% en poids et dans laquelle lesdits constituants chimiques peuvent être dosés par une détermination quantitative photométrique par absorption infrarouge, la détermination de la teneur en constituants dans la dispersion par cette détermination photométrique et la mise en corrélation de la détermination ainsi obtenue avec une détermination quantitative des constituants dosés par une méthode de référence.

2. Procédé selon la revendication 1, dans lequel les constituants chimiques déterminés comprennent graisse et protéine.

3. Procédé selon l'une quelconque des revendications 1 ou 2, dans lequel l'échantillon est un échantillon solide ou semi-solide choisi dans le groupe constitué par les aliments, les aliments pour animaux et les matières premières pour aliments et aliments pour animaux.

4. Procédé selon l'une quelconque des revendications précédentes, dans lequel le traitement mécanique destiné à diviser finement l'échantillon est réalisé sur une boue de l'échantillon de concentration en solides égale au plus à 30 g pour 100 ml, typiquement 2—20 g pour 100 ml.

5. Procédé selon l'une quelconque des revendications précédentes, dans lequel la dispersion soumise à la détermination photométrique a une granulométrie inférieure à 6 $\mu$m environ.

6. Procédé selon la revendication 5, dans lequel la granulométrie est égale ou inférieure à 1 $\mu$m environ.

7. Procédé selon la revendication 5 ou 6, dans lequel la réduction en une granulométrie maximale déterminée est réalisée seulement par la combinaison de la subdivision mécanique et du traitement avec le réactif de dispersion et sans réaliser de séparation de la dispersion.

8. Procédé selon la revendication 5 ou 6, dans lequel les particules de granulométrie supérieure ou égale à 6 $\mu$m sont séparées de la dispersion avant la détermination photométrique.

9. Procédé selon la revendication 8, dans lequel 10% au plus, de préférence 5% au plus, du constituant ou des constituants à déterminer sont éliminés par séparation.

10. Procédé selon la revendication 8 ou 9, dans lequel la séparation est réalisée par centrifugation à raison d'environ 2000 g×10 mn.

11. Procédé selon la revendication 8 ou 9, dans lequel la séparation est réalisée par filtration à travers un filtre permettant le passage des particules de 5 $\mu$m au plus.

12. Procédé selon l'une quelconque des revendications précédentes, dans lequel le traitement mécanique est réalisé dans un broyeur possédant une enceinte cylindrique fermée dans laquelle est montée coulissante une pièce cylindrique lisse de diamètre sensiblement plus faible que celui de l'enceinte pour se déplacer sensiblement selon un mouvement de va-et-vient d'amplitude prédéterminée.

13. Procédé selon la revendication 12, dans lequel l'amplitude est d'environ 5 mm, la fréquence du mouvement d'environ 3000 par mn, la durée du traitement d'environ 4 mn et le poids de la pièce cylindrique d'environ 400 g.

14. Procédé selon l'une quelconque des revendications précédentes, dans lequel le réactif de dispersion est une solution aqueuse.

15. Procédé selon la revendication 14, dans lequel la solution aqueuse est une solution basique.

16. Procédé selon la revendication 15, dans lequel le pH de la solution est au moins égal à 9.

17. Procédé selon la revendication 16, dans lequel le pH de la solution est de 12—14.

18. Procédé selon l'une quelconque des revendications précédentes, dans lequel l'agent de dispersion est une solution d'hydroxyde alcalin telle qu'une solution de NaOH environ 0,5 M.

19. Procédé selon l'une quelconque des revendications 1 à 13, dans lequel l'agent de dispersion est un réactif complexable avec les protéines pour former une solution ou une solution colloïdale.

20. Procédé selon la revendication 19, dans lequel le réactif complexable est une solution basique contenant du cuivre.

21. Procédé selon la revendication 20, dans lequel la solution basique contenant du cuivre est une solution basique d'un complexe de cuivre avec un composant choisi dans le groupe constitué par les amines, l'ammoniac, les tartrates et l'éthylèneglycol.

22. Procédé selon la revendication 19, dans lequel le réactif basique complexable est une solution basique contenant un complexe dissolvant les protéines choisi dans le groupe constitué par les complexes de nickel, les complexes de mercure, les complexes de cadmium, les complexes de zinc et les complexes de cobalt.

23. Procédé selon l'une quelconque des revendications précédentes, dans lequel le réactif de dispersion contient un émulsifiant.

24. Procédé selon l'une quelconque des revendications précédentes, dans lequel la détermina-

11

tion quantitative de la teneur en graisse et en protéine de l'échantillon basée sur la mesure photométrique de la transmission IR est réalisée en établissant la relation entre la mesure de la transmission IR et les déterminations quantitatives faites sur le même matériau que celui qui constitue les échantillons au moyen de méthodes standard.

25. Procédé selon la revendication 24, dans lequel la méthode standard avec laquelle on établit une relation est la méthode de Soxhlet ou Foss-Let pour la détermination des graisses et la méthode de Kjeldahl ou Kjelfoss pour la détermination des protéines.

26. Procédé selon l'une quelconque des revendications précédentes, dans lequel la température de l'échantillon lorsqu'il est traité avec le réactif de dispersion est d'au moins 40°C.

## Patentansprüche

1. Verfahren zur quantitativen Bestimmung des Gehalts von wenigstens zwei chemischen Bestandteilen, wie Fett und Protein, in einer festen oder halbfesten Probe eines proteinhaltigen, nicht auf Milch basierenden Produkts, umfassend

— das Umwandeln der Probe in eine dispergierte Form durch Behandlung mit einem Dispergiermittel, verbunden mit mechanischer Unterteilung, um eine Dispersion zu erhalten, in der der Proteingehalt bis zu 20 Gew.-% beträgt und in der die chemischen Bestandteile durch eine quantitative photometrische Infrarot-Absorptionsstimmung (IR-Spektroskopie) bestimmt werden können,

— das Bestimmen des Gehalts der Bestandteile in der Dispersion durch die photometrische Bestimmung, und

— in Korrelation bringen der so erhaltenen Bestimmung mit einer quantitativen Bestimmung der Bestandteile, wie bestimmt durch ein Referenzverfahren.

2. Verfahren nach Anspruch 1, wobei die bestimmten chemischen Bestandteile Fett und Protein umfassen.

3. Verfahren nach Anspruch 1 oder 2, wobei die Probe eine feste oder halbfeste Probe ist, gewählt aus der Gruppe, bestehend aus Lebensmitteln, Tierfutter und Rohstoffen für Lebensmittel und Tierfutter.

4. Verfahren nach mindestens einem der vorhergehenden Ansprüche, wobei die mechanische Behandlung zur feinen Zerteilung der Probe mit einer Aufschlämmung der Probe durchgeführt wird, die eine Feststoffkonzentration von höchstens 30 g pro 100 ml, typischerweise 2—20 g pro 100 ml, besitzt.

5. Verfahren nach mindestens einem der vorhergehenden Ansprüche, wobei die Dispersion, die der photometrischen Bestimmung ausgesetzt wird, eine Teilchengröße von weniger als etwa 6 $\mu$m besitzt.

6. Verfahren nach Anspruch 5, wobei die Teilchengröße etwa 1 $\mu$m oder weniger beträgt.

7. Verfahren nach Anspruch 5 oder 6, wobei die Verringerung auf die angegebene maximale Teilchengröße allein durch die Kombination der mechanischen Unterteilung und Behandlung mit dem Dispergiermittel und ohne Durchführung irgendeiner Trennung mit der Dispersion durchgeführt wird.

8. Verfahren nach Anspruch 5 oder 6, wobei die Teilchen, deren Größe 6 $\mu$m oder mehr beträgt, vor der photometrischen Bestimmung von der Dispersion getrennt werden.

9. Verfahren nach Anspruch 8, wobei höchstens 10%, vorzugsweise höchstens 5%, des zu bestimmenden Bestandteils oder der zu bestimmenden Bestandteile durch die Trennung entfernt werden.

10. Verfahren nach Anspruch 8 oder 9, wobei die Trennung durch Zentrifugieren bis zu einem Grad, entsprechend etwa 2 000 g×10 min, durchgeführt wird.

11. Verfahren nach Anspruch 8 oder 9, wobei die Trennung durch Filtration durch einen Filter, der das Passieren von Teilchen mit höchstens 5 $\mu$m erlaubt, durchgeführt wird.

12. Verfahren nach mindestens einem der vorhergehenden Ansprüche, wobei die mechanische Behandlung in einer Mahl- bzw. Zerkleinerungsvorrichtung mit einer geschlossenen, zylindrischen Kammer durchgeführt wird, in der ein glatter Zylinderkörper mit wesentlich geringerem Durchmesser als die Kammer verschiebbar zum Zwecke einer überwiegenden Hin- und Herbewegung mit vorbestimmter Hublänge angebracht ist.

13. Verfahren nach Anspruch 12, wobei die Hublänge etwa 5 mm, die Hubfrequenz etwa 3 000 pro Minute, die Behandlungszeit etwa 4 Minuten und das Gewicht des Zylinderkörpers etwa 400 g beträgt.

14. Verfahren nach mindestens einem der vorhergehenden Ansprüche, wobei das Dispergiermittel eine wäßrige Lösung ist.

15. Verfahren nach Anspruch 14, wobei die wäßrige Lösung eine basische Lösung ist.

16. Verfahren nach Anspruch 15, wobei der pH der Lösung wenigstens 9 ist.

17. Verfahren nach Anspruch 16, wobei der pH der Lösung 12—14 ist.

**0 046 783**

18. Verfahren nach mindestens einem der vorhergehenden Ansprüche, wobei das Dispergiermittel eine Alkalihydroxidlösung, wie eine etwa 0,5 M NaOH-Lösung, ist.

19. Verfahren nach mindestens einem der Ansprüche 1 bis 13, wobei das Dispergiermittel ein Reagens ist, das mit Proteinen einen Komplex bildet, um eine Lösung oder eine kolloide Lösung zu bilden.

20. Verfahren nach Anspruch 19, wobei das komplexbildende Reagens eine basische, kupferhaltige Lösung ist.

21. Verfahren nach Anspruch 20, wobei die basische Kupferlösung eine basische Lösung eines Kupferkomplexes mit einer Verbindung, gewählt aus der Gruppe, bestehend aus Aminen, Ammoniak, Tartraten und Ethylenglykol, ist.

22. Verfahren nach Anspruch 19, wobei das komplexbildende, basische Reagens eine basische Lösung ist, die einen proteinlösenden Komplex, gewählt aus der Gruppe, bestehend aus Nickelkomplexen, Quecksilberkomplexen, Cadmiumkomplexen, Zinkkomplexen und Kobaltkomplexen, enthält.

23. Verfahren nach mindestens einem der vorhergehenden Ansprüche, wobei das Dispergiermittel einen Emulgator enthält.

24. Verfahren nach mindestens einem der vorhergehenden Ansprüche, wobei die quantitative Bestimmung des Gehalts an Fett und Protein in der Probe auf der Basis der photometrischen IR-Durchlässigkeitsmessung durchgeführt wird, indem die IR-Durchlässigkeitsaufzeichnung zu quantitativen Bestimmungen, die mit demselben Material als Probenbestandteile mittels Standardverfahren gemacht wurden, in Bezug gesetzt wird.

25. Verfahren nach Anspruch 24, wobei das Standardverfahren, auf das Bezug genommen wird, das Soxhlet- oder Foss-Let-Verfahren für die Fettbestimmung und das Kjeldahl- oder Kjelfoss-Verfahren für die Proteinbestimmung ist.

26. Verfahren nach mindestens einem der vorhergehenden Ansprüche, wobei die Temperatur der Probe bei der Behandlung mit dem Dispergiermittel wenigstens 40°C beträgt.